# EUROPÄISCHE PATENTSCHRIFT

(11) **EP 1 128 833 B1**
(45) Veröffentlichungstag und Bekanntmachung des Hinweises auf die Patenterteilung: **25.04.2007**
(21) Anmeldenummer: 99955963.6
(22) Anmeldetag: 09.11.1999
(51) Int. Cl.: A61K 31/573, A61K 31/4184, A61P 25/30, A61P 25/36

(54) **VERWENDUNG VON AGONISTEN DER CORTICOSTEROIDREZEPTOREN UND EINER SUCHTDROGE ZUR SUCHTBEHANDLUNG**
USE OF AGONISTS OF CORTICOSTEROID RECEPTORS AND A DRUG TO TREAT ADDICTION
UTILISATION D'AGONISTES DES RECEPTEURS CORTICOSTEROIDES ET D'UNE DROGUE POUR LE TRAITEMENT DE TOXICOMANIE

(30) Priorität: 10.11.1998 EP 98121338
(43) Veröffentlichungstag der Anmeldung: 05.09.2001
(73) Patentinhaber: CURAMENTIS LTD., Nottingham NG1 5EL, Nottinghamshire (GB)
(72) Erfinder: Wolffgramm, Jochen, 72127 Mähringen (DE)
(74) Vertreter: Vossius & Partner
(86) Internationale Anmeldenummer: PCT/EP1999/008598
(87) Internationale Veröffentlichungsnummer: WO 2000/027400

(56) Entgegenhaltungen:
- WO-A-97/03677
- WO-A-98/42275
- MONTGOMERY, STEPHEN P. ET AL: "Cyclophosphamide and cortisol reduce the severity of morphine withdrawal" INT. J. IMMUNOPHARMACOL. (1987), 9(4), 453-7 CODEN: IJIMDS;ISSN: 0192-0561, XP002100181
- CAPASSO, A. ET AL: "Dexamethasone selective inhibition of acute opioid physical dependence in isolated tissues" J. PHARMACOL. EXP. THER. (1996), 276(2), 743-51 CODEN: JPETAB;ISSN: 0022-3565, XP002100182
- SZE, PAUL Y.: "The permissive role of glucocorticoids in the development of ethanol dependence and tolerance" DRUG ALCOHOL DEPEND. (1977), 2(5-6), 381-96 CODEN: DADEDV, XP002100183
- CAPASSO, ANNA ET AL: "Dexamethasone pretreatment reduces the psychomotor stimulant effects induced by cocaine and amphetamine in mice" PROG. NEURO-PSYCHOPHARMACOL. BIOL. PSYCHIATRY (1995), 19(6), 1063-79 CODEN: PNPPD7;ISSN: 0278-5846,1995, XP002100184
- ALBRECHT K. ET AL: "[Diagnosis and therapy of acute drug emergencies]. DIAGNOSTIK UND THERAPIE VON AKUTEN DROGENNOTFALLEN." ZEITSCHRIFT FUR ARZTLICHE FORTBILDUNG, (1992) 86/14 (701-707). ISSN: 0044-2178 CODEN: ZAFBAX, XP002100185 Germany
- MANTSCH J R ET AL: "Corticosterone facilitates the acquisition of cocaine self-administration in rats: opposite effects of the type II glucocorticoid receptor agonist dexamethasone." JOURNAL OF PHARMACOLOGY AND EXPERIMENTAL THERAPEUTICS, (1998 OCT) 287 (1) 72-80. JOURNAL CODE: JP3. ISSN: 0022-3565., XP002100186 United States
- CAPASSO, ANNA ET AL: "Dexamethasone inhibition of acute opioid physical dependence in vitro is reverted by anti-lipocortin-1 and mimicked by anti-type II extracellular PLA2 antibodies" LIFE SCI. (1997), 61(10), PL127-PL134 CODEN: LIFSAK;ISSN: 0024-3205,1997, XP002100187
- REDDY, D. S. ET AL: "Neurosteroid coadministration prevents development of tolerance and augments recovery from benzodiazepine withdrawal anxiety and hyperactivity in mice" METHODS FIND. EXP. CLIN. PHARMACOL. (1997), 19(6), 395-405 CODEN: MFEPDX;ISSN: 0379-0355, XP002100188

## Beschreibung

Gegenstand der vorliegenden Erfindung ist die Verwendung von Agonisten der Corticosteroidrezeptoren, ausgewählt aus den Verbindungen Cortisol, Cortison, Cortisonacetat, Corticosteron, Prednisolon, Prednison, Prednyliden, Methylprednisolon, Triamcinolon, Betamethason, Dexamethason, Paramethason, Fluoconolon, Deflazacort, Cloprednol und Fludrocortison oder Kombinationen davon, und einer Suchtdroge, ausgewählt aus der Gruppe umfassend Opiate, Psychostimulantien, Halluzinogene und Entactogene, insbesondere Amphetamine, LSD und MDMA (Ecstasy), Nikotin, Cannabinoide, Kokain (inklusive "Crack") zur Herstellung eines Arzneimittels zur Therapie der durch die Suchtdroge ausgelösten psychischen Abhängigkeit nach erfoltem Entzung.

Alkohol- und Drogensucht gelten bis heute als nicht heilbar. Alle Therapieprogramme, auch die neuen Ansätze einer "anti-craving"-Pharmakotherapie könnten den süchtigen Patienten zwar in seinem Willen unterstützen, nach einem Entzug nicht wieder rückfällig zu werden, sie können jedoch den Kern der Erkrankung - den latenten Kontrollverlust über die Drogeneinnahme - nicht rückgängig machen. Daher ist auch Jahre nach dem Entzug die Rückfallgefahr weiterhin hoch.

Alkohol- und Drogensucht (häufig auch als "Abhängigkeit" bezeichnet) ist eine psychische Erkrankung, welche sich in einer zwanghaft gesteigerten Selbstzufuhr der Suchtdroge ausdrückt. Der süchtige Konsument ist nicht mehr in der Lage, seine Drogenzufuhr zu regulieren, an Begleitbedingungen (z. B. die aktuelle soziale Situation) anzupassen und gegenüber anderen Verhaltensalternativen abzuwägen. (American Psychiatric Association, 1994). Der "Kontrollverlust" geht, wenn er einmal eingetreten ist, nur äußerst langsam zurück, in vielen Fällen scheint er sogar spontan nicht mehr reversibel zu sein (Sobell et al., 1993). Dieser "Reversibilitätsverlust" wird statistisch an den äußerst hohen Rückfallquoten selbst nach langen Abstinenzzeiten sichtbar. Bei "follow-up"-Studien nach Entgiftung und anschließender Therapie von Alkoholsüchtigen findet man einen Anteil von gerade einem Viertel bis maximal einem Drittel der Suchtpatienten, die dauerhaft abstinent geblieben sind (Süß, 1995; Veltrup et al., 1995). Spontane Heilungserfolge und höhere Quoten an langfristig abstinenten Patienten gehen meistens darauf zurück, dass - bedingt durch die Einschlusskriterien der betreffenden Studie - nicht nur eindeutig abhängige Patienten, sondern auch "Problemtrinker", also zwar exzessive, aber kontrollierte Alkoholkonsumenten in die Untersuchung einbezogen wurden (vgl. Stetter und Axmann-Kremar, 1996; Wieser und Kunad, 1965). Bei anderen Drogen (Opiate, Kokain, Amphetaminderivate) liegen weniger verlässliche "follow-up"-Daten vor. Für Opiatsucht ist jedoch in der Regel von einer noch schlechteren Heilungsprognose auszugehen als für Alkoholsucht (vgl. Roch et al., 1992).

Ein Vergleich verschiedener Therapieansätze und Behandlungsfaktoren (vgl. Küfner, 1997) wird dadurch erschwert, dass sich verschiedene Therapieeinrichtungen und -programme nicht nur in ihren Randbedingungen (Einschlusskriterien, offene oder geschlossene Therapie, Behandlungsdauer, Nachbehandlungsdauer, Abbruchkriterien, Dokumentation etc.) unterscheiden, sondern auch darin, dass das Therapiekriterium nicht einheitlich definiert ist. Die Spanne reicht von totaler Abstinenz über eine weitgehende Abstinenz mit wenigen, tolerierten Rückfällen bis zu einer moderaten, kontrollierten Substanzeinnahme. Wäre letztere in größerem Umfang möglich, so bedeutete dies den Schritt zurück von Kontrollverlust zum kontrollierten Konsum. Dies ist aber nur sehr selten der Fall. Rist (1996) verweist bezüglich Alkoholismustherapien auf eine Metaanalyse von Süß (1995) und die "VDR"-Studie (Küfner und Feuerlein, 1989), nach denen der Anteil nicht dauerhaft abstinenter, aber gebesserter Patienten vergleichsweise gering ist. Danach hat ein latent Süchtiger im Grunde genommen nur die Wahl zwischen einem Rückfall in die Sucht und einer Fortführung der Abstinenz unter ständigem Kampf gegen den Rückfall.

Die gängigen Therapieformen unterstützen den Patienten allenfalls in diesem Kampf. Da Anlässe für einen Rückfall häufig umfeldbedingt sind, ist das Hauptziel vieler psycho- und verhaltenstherapeutischen Ansätze eine psychische und soziale Konsolidierung des Patienten. Dazu kommen Aufklärung und Information, die ein kompetentes Umgehen des Suchtkranken mit seiner Erkrankung zum Ziel haben sowie zum Teil ein spezielles Bewältigungs-Training ("coping skills", Rist, 1996). Konditionierungs- und Aversionsverfahren sollen gelernte Zusammenhänge (Reiz-Reaktions-Beziehungen, die mit Substanzzufuhr in Verbindung stehen) brechen bzw. neue, aversive Assoziationen mit der Alkohol- und Drogeneinnahme schaffen.

Medikamentöse Behandlungsformen sind im Regelfall Ergänzungen einer Psycho- bzw. Verhaltenstherapie, zum Teil werden sie aber auch ohne flankierende Maßnahmen eingesetzt. Es lassen sich grob drei Einsatzbereiche unterscheiden: (a) Substitutionsbehandlung, (b) Anti-Reward-Therapien und (c) Anti-Craving-Therapien. Substitutionsbehandlungen sind bei einer Opiatsucht seit Jahren eingeführt (Finkbeiner et al., 1996; Bühringer et al., 1997). Anstelle der Suchtdroge (meist Heroin) nimmt der Süchtige die pharmakodynamisch gleichartige Substitirtionssubstanz (meist Methadon) zu sich. Eine Sucht-"Therapie" stellt dieses Konzept nicht dar, denn nach (ausschleichendem) Absetzen des Substituenten kehrt das Bedürfnis nach der Originaldroge zurück, oft ist es auch während der Substitution noch latent vorhanden. Der Nutzen einer Substitution liegt eher bei sozialen Faktoren (Re-Integration des Suchtkranken), in einer Entkriminalisierung der Drogenszene und in einer Reduktion von Morbidität und Mortalität.

Anti-Reward-Behandlungen mit Pharmaka lehnten sich früher an die A-versions-Strategie an (Beispiel: Disulfiram-Behandlung des Alkoholismus). Dieses Vorgehensweise war in Europa lange umstritten, sie ist aber kürzlich wieder aufgegriffen worden (OLITA- bzw. ALITA-Programm: Ehrenreich et al., 1997). Im Vergleich zu herkömmlichen Therapieprogrammen unterscheidet sich das OLITA/ALITA-Konzept durch eine intensive ambulante Langzeitbehandlung (zweijährige, tägliche Einbestellung der Alkoholismuspatienten zu Gesprächskontakten bei gleichzeitiger Verabreichung von Acetaldeyd-Dehydrogenase-Hemmstoffen zur Induktion einer Alkoholunverträglichkeit). Die Autoren der Studie berichten über einen ca. 50% der Patienten umfassenden Therapieerfolg (fortdauernde Abstinenz), damit wäre das Behandlungsprogamm erfolgreicher als übliche Therapieansätze. Längerfristige follow-up-Studien stehen allerdings noch aus. Eine größere multizentrische Studie befindet sich momentan in der Planungsphase (mündliche Mitteilung).

Hoffnungen werden zur Zeit auf eine mittelfristige Blockade der zentralnervösen opioidergen Übertragung durch Opioid-Antagonisten Naltrexon gesetzt. Bei der Opiatverabreichung verspürt der zuvor Naltrexonbehandelte Suchtpatient keine Wirkung, also auch keine Belohnungseffekt. Dies senkt das Rückfallrisiko. Das Problem bleibt die "Compliance" des Patienten, also seine Bereitschaft, das Medikament zu akzeptieren. Daher ist der Nutzen einer Naltrexon-Therapie umstritten. Im kurz- bis mittelfristigen Zeitbereich scheint eine Naltrexon-Therapie wirksam zu sein, d. h. sie kann die Zahl der Rückfälle senken und auch deren Schweregrad günstig beeinflussen. Dies gilt insbesondere auch für die Behandlung von Alkoholikern (Mann und Mundle, 1996). Anhaltende Effekte über das Ende der Pharmakatherapie hinaus sind bisher nicht beschrieben worden.

Anti-Craving-Medikationen sollen laut Definition das zwanghafte Verlangen des Suchtkranken nach seiner Droge ("craving") reduzieren. Es gibt eine Fülle von derartigen Ansätzen, die sich nicht einem gemeinsamen Prinzip unterordnen lassen. Auch die oben genannte Naltrexon-Therapie wird zum Beispiel als Anti-Craving-Strategie eingesetzt, obwohl sie pharmakologisch vermutlich eher auf einer Wirkungsblockade als auf einer Motivationsbeeinflussung beruht. Grundsätzlich scheinen die Wirkungen und Wirkmechanismen der putativen Anti-Craving-Pharmaka heterogen zu sein. Eine Zusammenstellung von Soyka (1997) zählt Glutamat-Modulatoren, Glutamat-Antagonisten, Opiat-Antagonisten, Dopamin-Agonisten, Dopamin-Antagonisten, Serotonin-Reuptake-Hemmer, Serotonin-Antagonisten und MAO-Hemmer auf. Neben dem bereits genannten Naltrexon, bei dem eine positive Wirkung wahrscheinlich ist, hat sich bisher nur der Glutamat-Modulator Acamprosat in europaweiten multizentrischen klinischen Tests mit Alkoholkranken als wirksam erwiesen. Im deutschen Teil der Studie (Sass et al., 1996) waren nach 48-wöchiger Behandlung am Studienende noch 42% der Patienten der Verum-Gruppe abstinent, bei der Placebogruppe waren es nur 20%. In Österreich waren es 30% der Acamprosat-behandelten und 21% der Placebogruppe (Wirtworth et al., 1996). Über Langzeiteffekte nach Absetzen des Präparates liegen noch keine statistisch aussagekräftigen Daten vor. Nimmt man alle Studien zusammen, so bewirkt Acamprosat - zumindest während des Behandlungszeitraums - etwa eine Verdoppelung der Aussicht, nach etwa einjähriger Behandlung abstinent zu bleiben. Dies ist ein nicht zu unterschätzender Erfolg. Dennoch bleibt festzuhalten, dass im europäischen Durchschnitt trotz Acamprosat-Behandlung ca. 70% der Suchtpatienten das Therapieziel einer dauernden Abstinenz nicht erreichten (bei Placebo-Behandlung waren es 85%). Damit kann selbst das bis heute wirksamste Sucht-Therapeutikum nur bei einem relativ geringen Anteil der Suchtkranken erfolgreich eingesetzt werden.

Der hohe Anteil von 60 bis 90% Therapiefehfschlägen zeigt, dass die heute etablierten Formen der Psycho-, Verhaltens- und Pharmakotherapie die Krankheit Sucht entweder gar nicht oder nur unzureichend heilen können. Zusätzlich ist zu bedenken, dass viele der abstinent gebliebenen Patienten täglich mit Rückfallgedanken zu kämpfen haben und sich in Selbsthilfegruppen, wie den Anonymen Alkoholikern, organisieren, um sich gegenseitig bei ihrer Auseinandersetzung mit der latenten Sucht zu unterstützen (vgl. Schwoon. 1996), Die Zahl Wirklich geheilten, d. h. von ihrem Kontrollverlust befreiten Suchtpatienten dürfte daher noch deutlich niedriger liegen, als es die Rückfallstatistiken belegen.

Wünschenswert ist es mithin ein Behandlungsverfahren anzugeben, mit dem ein Kontrollverlust wieder aufgehoben werden kann, so dass der zuvor süchtige Patient wieder in den status quo ante zurückversetzt wird. Um dieses Ziel zu erreichen, hat sich die vorliegende Erfindung die Aufgabe gestellt, Substanzen anzugeben, die eine medikamentöse Behandlung einer Suchterkrankung ermöglichen. Diese Medikamente sollen in einer kausal wirkenden Therapie einsetzbar sein.

Diese Aufgabe wird gelöst durch die Verwendung von Agonisten der Corticosteroidrezeptoren, ausgewählt aus den Verbindungen Cortisol, Cortison, Cortisonacetat, Corticosteron, Prednisolon, Prednison, Prednyliden, Methylprednisolon, Triamcinolon, Betamethason, Dexamethason, Paramethason, Fluoconolon, Deflazacort, Cloprednol und Fludrocortison oder Kombinationen davon, und einer Suchtdroge, ausgewählt aus der Gruppe umfassend Opiate, Psychostimulantien, Halluzinogene und Entactogene, insbesondere Amphetamine, LSD und MDMA (Ecstasy), Nikotin, Cannabinoide, Kokain (inklusive "Crack") zur Herstellung eines Arzneimittels zur Therapie der durch die Suchtdroge ausgelösten psychischen Abhängigkeit nach erfoltem Entzung.

Als Suchtdroge kommt beispielsweise ein Opiat in Frage oder aber diejenige Droge, von der der Patient abhängig ist.

Als Stand der Technik sind weiterhin zu nennen: MONTGOMERY, STEPHEN P. EI AL, Cyclophosphamide and cortisol reduce the severity of morphine withdrawal, INT. J. IMMUNOPHARMACOL. (1987), 9(4),453-7, CAPASSO, A. EI AL: ,Dexametbasone selective inhibition of acute opioid physical dependence in isolated tissues, PHARMACOL. EXP. THER. (1996), 276 SZE, PAUL Y. : The permissive role of 1-3 glucocorticoids in the development of ethanol dependence and tolerance, DRUG ALGOHOL DEPEND. (1977), 2(5-6), 381-96, CAPASSO, ANNA ET AL: Dexamethasone 1-3 pretreatment reduces the psychomotor stimulant effects induced by cocaine and amphetamine in mice, PROG. NEURO-PSYCHOPHARMACOL. BIOL. PSYCHIATRY (1995), 19(6), 1063-79, ALBRECHT K. ET AL: Diagnosis and therapy of acute drug emergencies, DIAGNOSTIK UND THERAPIE VON AKUTEN DROGENNOTFALLEN, ZEITSCHRIFT FUR ÄRZTLICHE FORTBILDUNG, (1992) 86/14 (701-707), MANTSCH J R ET AL: Corticosterone facilitates the acquisition of cocaine self-administration in rats: opposite effects of the type II glucocorticoid receptor agonist dexamethasone., JOURNAL OF PHARMACOLOGY AND EXPERIMENTAL THERAPEUTICS, (1998 OCT) 287 (1) 72-80, CAPASSO, ANNA ET AL: Dexamethasone 1-3 inhibition of acute opioid physical dependence in vitro is reverted by anti-lipocortin-1 and mimicked by anti-type II extracellular PLA2 antibodies, LIFE SCI. (1997), 61(10), PL127-PL134, REDDY, D. 5. ET AL: Neurosteroid 1-3 coadministration prevents development of tolerance and augments recovery from benzodiazepine withdrawal anxiety and hyperactivity in mice, METHODS FIND. EXP. CLIN. PHARMACOL. (1997), 19(6), 395-405 sowie WO - A - -97 03677.

Keine der Arbeiten offenbart die Heilung der Sucht oder eine nachhaltige Therapie einer manifesten Sucht. In einigen der Publikationen werden Akutwirkungen, Toleranzentwicklung, körperliche Abhängigkeit und Entzugssyndrome infolge von Drogeneinnahme beschrieben. Unter anderem hierauf wirken sich Corticoide offenbar aus. Ziel einer Therapie kann aber nicht die Behandlung eines Entzugs sein, sondern kann nur die Therapie einer manifestierten Sucht, d.h. einer psychischen Abhängigkeit bedeuten. Ein Entzug, also eine Entgiftung des körperlich von der Droge abhängigen Patienten sollte bereits vor der eigentlichen Suchtbehandlung stattgefunden haben. Bei einer solchen Entgiftung kommen verschiedene Medikamente zum Einsatz, darunter möglicherweise auch Corticosteroide, diese dienen jedoch nicht zur Suchtbehandlung.

Die Behandlung der psychischen Abhängigkeit (d.h. der Sucht) erfolgt vorzugsweise am bereits entgifteten Patienten, sie soll ihn von seiner unkontrollierbaren Drogengier befreien. Keine der Publikationen betrifft die Heilung psychischer Abhängigkeit.

Eine Suchtbehandlung mit einem Corticosteroid als Monosubstanz führt nicht zu einer Heilung. Entscheidend ist die Kombination mit der gleichzeitig/anschließend verabreichten Suchtdroge. Keine der genannten Publikationen setzt eine solche Kombination ein, um ein nachhaltiges, kausales therapeutisches Ziel zu erreichen. In keiner der Studien wurden süchtige Patienten oder Versuchstiere einer Kombinationsbehandlung unterzogen.

Im einzelnen ist zu den folgenden Publikationen Montgomery & Dafny, 1987, Capasso et al., 1996, Capasso et al, 1997,Reddy & Kulkarni,-1997anzumerken:

Die in den genannten Veröffentlichungen dargestellten Studien betreffen die Wirkungen von Corticosteroiden auf Toleranzentwicklung, körperliche Abhängigkeit und Entzugserscheinungen infolge von Drogeneinnahme. Beschrieben wird u.a. eine entzugserleichternde Wirkung von Corticosteroiden. Eine entzugsbegleitende Medikation mit dem Ziel einer Milderung der Entzugssymptomatik ist nicht Zweck der erfindungsgemäß vorgeschlagenen Pharmakotherapie. Diese setzt insbesondere erst nach Abklingen der Entzugssymptomatik ein und verfolgt das Ziel, den Süchtigen von Drogengier und vom Kontrollverlust zu heilen. Es muss hervorgehoben werden, dass körperliche Abhängigkeit (welche zum Entzugssyndrom führt) und psychische Abhängigkeit (Sucht) völlig unterschiedlich sind.

Sze, 1997 beschreibt anhand von Versuchstieren, die nach einer Operation keine körpereigenen Corticosteroide mehr bilden konnten, die Beteiligung dieser Hormone an der Ausprägung von Toleranz und körperlicher Abhängigkeit gegenüber Alkohol. Dies geschah durch eine Substitution der nun körperseits fehlenden durch von außen zugeführte Corticoidhormone. Die Arbeit hat nichts mit Sucht zu tun (z.B. erfolgte keine freiwillige, sondern eine erzwungene Alkoholzufuhr), sie zeigt - ähnlich wie die o.g. Publikationen -, dass Corticosteroide an der Ausprägung von Toleranz, körperlicher Abhängigkeit und Entzugssymptomatik beteiligt sind.

Capasso et al., 1995 zeigt, dass bestimmte Corticosteroide die akuten psychostimulierenden Wirkungen von Cocain und Amphetamin reduzieren können. Diese Wirkung hat möglicherweise im präventiven Bereich (d.h. während eines Cocain oder Amphetaminabusus) eine gewisse Bedeutung, sie steht jedoch in keinerlei Beziehung zu einer Suchttherapie an einem Patienten, der den Entzug bereits durchlaufen hat und von seiner psychischen Drogenabhängigkeit befreit werden soll. Außerdem handelt es sich - wie bei allen anderen Arbeiten auch um Wirkungen einer Corticosteroid-Monosubstanz, nicht um die erfindungsgemäße Kombination mit der Suchtdroge. Nur eine solche Kombination ist erfolgreich.

Albrecht & Lampe, 1992 beschreiben notfallmedizinische Maßnahmen nach einer akuten Drogenvergiftung beschrieben. Dabei werden nach klassischer Indikation (insbesondere zur Immunsuppression) auch Corticosteroide eingesetzt. Die Corticosteroidbehandlung dient weder einer Suchttherapie noch wird sie in Kombination mit der Suchtdroge eingesetzt. Sie entspricht vielmehr dem üblichen therapeutischen Einsatz dieser Stoffklasse.
Mantsch et al., 1998 zeigt, dass manche Corticosteroide das Erlernen einer Cocain-Selbstverabreichungsaufgabe durch Ratten erleichtern, andere es eher blockieren. Ähnlich wie in der zuvor zitierten Arbeit von Capasso et al. 1995 werden durch das Corticosteroid die Auswirkungen einer Drogeneinnahme (dort: auf die motorischen Effekte, hier: auf die Selbstverabreichung) moduliert. Damit wird eine von vielen Corticosteroidwirkungen beschrieben. Diese Wirkung hat nichts mit der erfindungsgemäßen Kombinationstherapie einer Sucht am Post-Entzugs-Patienten zu tun. Erfindungsgemäß wird das Corticosteroid als Sensibilisator eines Prägungsprozesses eingesetzt.

WO-A-97/03677 betrifft die Hemmung einer Unterklasse von Glutamat-Rezeptoren (NMDARezeptoren) durch Pregnenolonsulfat-Derivate beschrieben. Die Methode wird auch als einsetzbar bei akutem Entzugssyndrom angesehen. Bekanntermaßen werden einige Entzugssymptome über NMDA-Rezeptoren des Gehirns vermittelt. Erfindungsgemäß wird aber nicht der Entzug erleichtert, sondern die Sucht kausal therapiert. Weiterhin werden erfindungsgemäß nicht Antagonisten des NMDA-Rezeptors eingesetzt, sondern Agonisten der Corticosteroidrezeptoren in Verbindung mit der insbesondere forciert verabreichten Suchtdroge.

Bei Tierversuchen, die auf einem anerkannten Tiermodell der Sucht basieren (Review-Artikel: Wolffgramm und Heyne, 1995) wurde festgestellt, dass eine Gabe von Corticosteroiden erfolgreich Suchtverhalten korrigieren kann, wenn die Gabe von Corticosteroiden kombiniert wird mit einer vorzugsweise hochdosierten, vom Einnahmewunsch des süchtigen Individuums entkoppelten Gabe einer Suchtdroge oder ihres pharmakodynamischen Äquivalents. Dabei kann die Suchtdroge oder ihr pharmakodynamisches Äquivalent gleichzeitig oder etwas zeitversetzt zur Gabe der Corticosteroide verabreicht werden. Das zur Prüfung der Therapie eingesetzte Tiermodell (Ratte) ist der internationalen Fachwelt bekannt und gestattet eine Analyse der Suchtentwicklung ebenso, wie eine Erprobung neuer therapeutischer Ansätze.

Nach mehrmonatiger Substanzeinnahme (Alkohol, Opiat Amphetamin usw.) bei freier Wahl (kein Tier wird gezwungen, die Suchtdroge einzunehmen), entwickeln manche Ratten spontan eine Sucht. Sie verlieren die Kontrolle über die Drogeneinnahme und nehmen zum Beispiel auch geschmacklich vergällte Drogenlösungen zu sich, was nicht-süchtige Tiere stets vermeiden. Zwischen Sucht und Nicht-Sucht gibt es wahrscheinlich keine intermediären Zustände, dies ist im Tiermodell zumindest für die Opiat- und Amphetaminsucht nachgewiesen: Nach zwangsweiser (forcierter) Drogenzufuhr, eine drogenhaltige Lösung dient als einzige Trinkflüssigkeit, entwickelt sich zwar eine physische Abhängigkeit (Entzugssyndrom) aber kein Kontrollverlust, d. h. keine Sucht.

Überraschenderweise hat sich gezeigt, dass bei einer Gabe von Agonisten der Glucocorticosteroid- und/oder Mineralcorticosteroidrezeptoren, insbesondere Corticosteroiden in einem gewissen zeitlichen Zusammenhang mit der forcierten Verabreichung einer Suchtdroge die Ausbildung der Sucht in den Stand vor der Sucht zurückversetzt wird. Ist der Zustand einer Sucht einmal erreicht, so ist weder die alleinige Verabreichung eines Agonisten der Glucocorticosteroid- und/oder Mineralcorticosteroidrezeptoren, insbesondere Corticosteroid, noch die forcierte (erzwungene) Einnahme der Suchtdroge alleine dazu geeignet, diesen Zustand wieder rückgängig zu machen. Insbesondere eine simultane bzw. sukzessive Kombination aus dem Agonisten der Glucocorticosteroid- und/oder Mineralcorticosteroidrezeptoren, insbesondere dem Corticosteroid, und einer zwangsweise verabreichten Suchtdroge können den süchtigen Patienten in einen Zustand rückversetzen, der dem vor der Sucht entspricht. Dabei scheint dem Agonisten der Glucocorticosteroid- und/oder Mineralcorticosteroidrezeptoren, insbesondere dem Corticosteroid eine vorbereitende oder synchronisierende Rolle zuzukommen ("ready to imprint" bzw. "imprint now!"), während die hochdosierte Verabreichung der Suchtdroge die bisher bestehenden Verknüpfungen löst, durch neue ersetzt und dadurch das bestehende "Suchtgedächtnis" löscht.

Als Agonisten der Glucocorticosteroid- und/oder Mineraicorticosteroidrezeptoren, insbesondere Corticosteroide, werden erfindungsgemäß die körpereigenen Corticoidhormone eingesetzt ausgewollt aus Corticosteron, Cortison, als Cortisonacetat, oder Cortisol. Ebenfalls können synthetische Verbindungen, wie Prednisolon, Prednison, Prednyliden, Methylprednisolon, Triamcinoion, Betamethason, Dexamethason, Paramethason, Fluocortolon, Deflazacort, Cloprednol und Fludrocortison oder Kombinationen davon aufnehmen. Vorzugsweise werden Cortisol und Cortison bei der Gruppe der natürlichen Corticoidhormone eingesetzt, da hier bereits umfangreiche Erfahrungen mit Verabreichungen am Menschen vorliegen. Zur Substitutionsbehandlung werden im allgemeinen nicht nur solche Corticoidhormone eingesetzt, die Affinität zu Glucocorticoidrezeptoren (GR), sondern auch an Mineralcorticoidrezeptoren (MR) aufweisen. Handelt es sich bei einem der aufgezählten Präparate um einen reinen GR-Agonisten, wie Betamethason, ist es möglicherweise vorteilhaft, zusätzlich ein MRwirksames Mineralcorticoid, wie Fludrocortison zu verabreichen. Fludrocortison könnte, da es auch glucocorticoide Effekte ausübt, auch als Monosubstanz wirksam eingesetzt werden.

Erfindungsgemäß lassen sich alle substanzbezogenen Suchterkrankungen behandeln. Der Anteil der Agonisten der Glucocorticosteroid- und/oder Mineralcorticosteroidrezeptoren bei der Therapie bleibt jeweils gleich. Der zweite Anteil wird von einer forciert verabreichten Suchtdroge dargestellt. Vorzugsweise sollte dabei entweder die Suchtdroge, von der der Patient abhängig ist oder ein Opiat verabreicht werden. Neben einer Opiatabhängigkeit lassen sich demgemäß auch die Nikotinsucht (Tabakrauchen), Cannabinoidabhängigkeit (Haschisch, Marihuana), Psychostimulantien- und Entactogenabhängigkeiten, Kokainsucht inklusive "Crack", sowie polytoxikomane Süchte mit dem Gebrauch der sogenannten Suchtformen heilen. Gegebenenfalls müssten in den zwei letztgenannten Fällen medizinische Begleitprobleme geklärt werden, die mit einer hochdosierten, eventuell mehrwöchigen Zwangsverabreichung einer Suchtdroge verbunden wären. Erfindungsgemäß wichtig ist die Kombination aus einer Verabreichung des Agonisten der Glucocorticosteroid- und/oder Mineralcorticosteroidrezeptoren, insbesondere Corticosteroids, und einer Suchtdroge. Die Verabreicherung des Agonisten der Glucocorticosteroid- und/oder Mineralcorticosteroidrezeptoren, insbesondere Corticosteroids muss vor und/oder während der forcierten Suchtdrogenverabreichung erfolgen. Beide Substanzen werden in hohen, aber nicht gesundheitsgefährdenden Dosierungen eingesetzt. Folgende Möglichkeiten sind gegeben, wobei die Verabreichungsdauern pro Phase jeweils zwischen einigen Tagen und wenigen Wochen liegen:
- gemeinsame Gabe des Agonisten der Glucocorticosteroid- und/oder Mineralcorticosteroidrezeptoren, insbesondere Corticosteroids, und einer Suchtdroge,
- erst die Verabreichung des Agonisten der Glucocorticosteroid-und/oder Mineralcorticosteroidrezeptoren, insbesondere des Corticosteroids, unmittelbar darauf die forcierte Verabreichung einer Suchtdroge,
- erst die Verabreichung des Agonisten der Glucocorticosteroid-und/oder Mineralcorticosteroidrezeptoren, insbesondere des Corticosteroids, unmittelbar darauf die gemeinsame Verabreichung von Corticosteroid und Suchtdroge,
- erst die gemeinsame Gabe von Agonisten der Glucocorticosteroid-und/oder Mineralcorticosteroidrezeptoren, insbesondere Corticosteroid, und Suchtdroge unmittelbar danach erfolgt die forcierte Verabreichung einer Suchtdroge ohne Agonisten der Glucocorticosteroid- und/oder Mineralcorticosteroidrezeptoren, insbesondere Corticosteroid,
- erst die Verabreichung des Agonisten der Glucocorticosteroid-und/oder Mineralcorticosteroidrezeptoren, insbesondere des Corticosteroids, danach die gemeinsame Verabreichung von Suchtdroge und Agonisten der Glucocorticosteroid-und/oder Mineralcorticosteroidrezeptoren, insbesondere Corticosteroid, danach die forcierte Verabreichung von Suchtdroge ohne Gabe des Agonisten der Glucocorticosteroid- und/oder Mineralcorticosteroidrezeptoren, insbesondere des Corticosteroids.

Neben der Verabreichung eines einzelnen Agonisten der Glucocorticosteroid- und/oder Mineralcorticosteroidrezeptoren, insbesondere Corticosteroids, kommt auch die Gabe von Kombinationen verschiedener Agonisten der Glucocorticosteroid- und/oder Mineralcorticosteroidrezeptoren, insbesondere Corticosteriode, in Frage. Bei Vorliegen einer Polytoxikomanie (Abhängigkeit von mehreren Drogen gleichzeitig) wäre sowohl eine gleichzeitige als auch eine sequentielle Behandlung möglich. Da bei einer sequentiellen Therapie zum einen zusätzliche Risiken entstünden und zum anderen ein Wirksamkeitsverlust bei mehrfacher Umprägung auftreten könnte, erscheint ein einmaliger Therapieansatz vorteilhaft. Hierbei könnte entweder die Hauptdroge eventuell in Kombination mit häufig eingesetzten Begleitsubstanzen bei der forcierten Verabreichung eingesetzt werden oder es könnte eine forcierte Opiatverabreichung erfolgen. Kombinationen von Agonisten der Glucocorticosteroid- und/oder Mineralcorticosteroidrezeptoren, insbesondere Corticosteroid, und Suchtdroge werden erfindungsgemäß so verstanden, dass entweder ein oder mehrere Agonisten der Glucocorticosteroid- und/oder Mineralcorticosteroidrezeptoren, insbesondere Corticosteroid(e), zusammen mit einer Suchtdroge in einer üblichen pharmazeutischen Formulierung zusammengemischt und dem Süchtigen dadurch gleichzeitig verabreicht werden. Es ist aber ebenfalls möglich, jeweils die Stoffe getrennt voneinander z. B. in galenischen Zubereitungen zu formulieren und dem Patienten voneinander getrennt zu geben. Dies hat beispielsweise den Vorteil, daß die Suchtdrogen oral und die Agonisten der Glucocorticosteroid- und/oder Mineraicorticosteroidrezeptoren, insbesondere Corticosteroide, parenteral gegeben werden könnten. Sofern jedoch eine orale oder andersartige Verabreichung für beide Komponenten des erfindungsgemäßen Arzneimittels möglich ist, kann es vorteilhaft sein, die Komponenten auch jeweils in einer Formulierung zu verabreichen.

Wird bei der Suchtbehandlung lediglich eine zweiphasige Behandlung anzustreben sein, in der zunächst der Agonist der Glucocorticosteroidund/oder Mineralcorticosteroidrezeptoren, insbesondere das Corticosteroid verabreicht wird, gefolgt von einer Kombination von Agonisten der Glucocorticosterold- und/oder Mineralcorticosteroidrezeptoren, insbesondere Corticosteriod, Suchtdroge so kann es vorteilhaft sein, die Verabreichung derart zu gestalten, daß beispielsweise eine Darreichungsform, z. B. Blisterpackung, zunächst eine für die erste Phase ausreichende Menge an Agonisten der Glucocorticosteroid- und/oder Mineralcorticosteroidrezeptoren, insbesondere Corticosteroid, enthaltenden Formulierungen, beispielsweise Tabletten oder Suppositorien, enthält, gefolgt von einer entsprechenden Anzahl von Tabletten, die die Kombination von Agonisten der Glucocorticosteroidund/oder Mineralcorticosteroidrezeptoren, insbesondere Corticosteroid, und Suchtdroge. Gegebenenfalls kann dann, falls eine dreiphasige Behandlung erforderlich ist, eine entsprechende Anzahl von Suchtdroge-enthaltenden Formulierungen vorhanden sein. Diese Konfiguration einer Formulierung stellt ein Beispiel eines zur Verabreichung der erfindungsgemäßen Arzneimittel geeigneten Darreichungsform dar. Abänderungen der beschriebenen Konfigurationen liegen im fachmännischen Können und sind auf die jeweilige Behandlungssituation anpassbar.

Die Dosen der einzusetzenden Agonisten der Glucocorticosteroidund/oder Mineralcorticosteroidrezeptoren, insbesondere Corticosteroide, und einer Suchtdroge sind individuell an den Patienten anzupassen, insbesondere müssen gesundheitsgefährdende Überdosierung vermieden werden. Wie Dosisfindungen bei einem Patienten individuell durchgeführt werden, ist dem Therapeuten bekannt. Zu unterscheiden ist erfindungsgemäß grundsätzlich die Gabe von Startdosierungen zu Beginn der Therapie sowie Erhaltungsdosen bei Fortführung der Therapie nach Gabe der Startdosis des entsprechenden Agonisten der Glucocorticosteroid- und/oder Mineralcorticosteroidrezeptoren, insbesondere Corticosteroids. Dabei wird die Dosis des Agonisten der Glucocorticosteroid- und/oder Mineralcorticosteroidrezeptoren, insbesondere Corticosteroids, anfänglich hoch sein (Startdosierung) und auf eine Erhaltungsdosis reduziert werden. Diese erstere orientiert sich für die verschiedenen Agonisten der Glucocorticosteroid- und/oder Mineralcorticos'teroidrezeptoren, insbesondere Glücocorticoide, an der "Cushing-Schwelle", bei deren Überschreiten ein typisches Syndrom unerwünschter Nebenwirkungen oder Begleitwirkungen auftritt: Die nachfolgend eingeblendete Tabelle ergibt Abschätzungen von Dosierungsrichtwerten für eine(n) 60 bis 80 kg schweren Patienten(in) an.

| Substanz | Erhaltungsdosis | Startdosis (in Vielfachen der Erhaltungsdosis |
|---|---|---|
| **Körpereigene. Glukocorticoidhormone:** | | |
| Cortisol (Hydrocortison) | 30 - 50 mg/Tag | x 3 |
| Cortisonacetat | 40 - 60 mg/Tag | x 2 |
| Corticosteron | 30 - 50 mg/Tag | x 2 |
| **Synthetische Glukocorticoide:** | | |
| Prednisolon | 8-12 mg/Tag | x 8 |
| Prednison | 8-12 mg/Tag | x 8 |
| Prednyliden | 8 - 12 mg/Tag | x 8 |
| Methylprednisolon | 8 - 12 mg/Tag | x 5 |
| Triamcinolon | 6 - 9 mg/Tag | x 6 |
| Betamethason | 1 - 2 mg/Tag | x 2 |
| Dexamethason | 2 - 3 mg/Tag | x 5 |
| Paramethason | 3 - 5 mg/Tag | x 3 |
| Fluocortolon | 10 - 15 mg/Tag | x 8 |
| Deflazacort | 10 - 15 mg/Tag | x 6 |
| Cloprednol | 8 - 12 mg/Tag | x 5 |
| **Synthetisches Mineralcorticoid (evtl. als Additivum)** | | |
| Fludrocortison | 0,2 - 0,3 mg/Tag | x 2 |

Die Dosierungen bei der forcierten (vom Verabreichungswunsch des Patienten unabhängige) Verabreichung der Suchtdroge richtet sich nach der höchsterreichbaren Dosis, bei der noch keine schweren, möglicherweise gesundheitsgefährdenden Begleitwirkungen einer chronischen Verabreichung zu erwarten sind. Das Eintreten der letztgenannten kann vom Gesundheitszustand des Patienten zu Beginn der Therapie entscheidend abhängen (z. B. Leberfunktion, Drogentoleranz etc.). Bei Opiatgabe muss zum Beispiel mit A-temdepressionen gerechnet werden. Wenn sich gegen diesen Effekt eine Toleranz (Unempfindlichkeit) entwickelt hat, so ist eine Dosiserhöhung möglich. Stellvertretend für andere Drogen sollen hier einige Richtwerte für Dosierungen von Opiaten angegeben werden (entsprechende Werte z. B. von Nikotin oder Tetrahydrocannabinol orientieren sich an den betreffenden Dosisschwellen der unerwünschten Begleitwirkungen):

| | |
|---|---|
| Morphin-Retard | 30 - 60 mg/Tag |
| Codein | 30 - 60 mg/Tag |
| Dihydrocodein | 60 - 120 mg/Tag |
| Levomethadon | 5 - 15 mg/Tag |
| Tilidin | 50- 100 mg/Tag |

Die erfingungsgemäße Verwendung zur Behandlung von Suchterkrankungen ist gekennzeichnet durch eine Gabe von mindestens einem Agonisten der Glucocorticosteroid- und/oder Mineralcorticosteroidrezeptoren, insbesondere Corticosteroid, und einer Suchtdroge. Dabei erfolgt die Verabreichung des Agonisten der Glucocorticosteroid- und/oder Mineralcorticosteroidrezeptoren, insbesondere Corticosteroids vor und/oder während der forcierten Gabe der betreffenden Suchtdroge Wie bereits zuvor beschrieben, ergeben sich daraus 5 Möglichkeiten:
1. Agonisten der Glucocorticosteroid- und/oder Mineralcorticosteroidrezeptoren, insbesondere Corticosteroid->Kombination aus Agonisten der Glucocorticosteroid- und/oder Mineralcorticosteroidrezeptoren, insbesondere Corticosteroids + Suchtdroge->Suchtdroge
2. Agonisten der Glucocorticosteroid- und/oder Mineralcorticosteroidrezeptoren, insbesondere Corticosteroid→Kombination aus Agonisten der Glucocorticosteroid- und/oder Mineralcorticosteroidrezeptoren, insbesondere Corticosteroid + Suchtdroge
3. Agonisten der Glucocorticosteroid- und/oder Mineralcorticosteroidrezeptoren, insbesondere Corticosteroid→Suchtdroge
4. Kombination aus Agonisten der Glucocorticosteroid- und/oder Mineralcorticosteroidrezeptoren, insbesondere Corticosteroid + Suchtdroge->Suchtdroge
5. Kombination aus Agonisten der Glucocorticosteroid- und/oder Mineralcorticosteroidrezeptoren, insbesondere Corticosteroid + Suchtdroge
   1. Verwendung von Agonisten der Corticosteroidrezeptoren, ausgewählt aus den Verbindungen Cortisol, Cortison, Cortisonacetat, Corticosteron, Prednisolon, Prednison, Prednyliden, Methylprednisolon, Triamcinolon, Betamethason, Dexamethason, Paramethason, Fluocortolon, Deflazacort, Cloprednol und Fludrocortison oder Kombinationen davon, und einer Suchtdroge, ausgewählt aus der Gruppe umfassend Opiate, Psychostimulantien, Halluzinogene und Entactogene, insbesondere Amphetamine, LSD und MDMA (Ecstasy), Nikotin, Cannabinoide, Kokain (inklusive "Crack") zur Herstellung eines Arzneimittels zur Therapie der durch die Suchtdroge ausgelösten psychischen Abhängigkeit nach erfolgtem Entzug,
   2. Arzneimittel enthaltend einen Agonisten der Corticosteroidrezeptoren ausgewählt aus den Verbindungen Cortisol, Cortison, Cortisonacetat, Corticosteron, Prednisolon, Prednison, Prednyliden, Methylprednisolon, Triamcinolon, Betamethason, Dexamethason, Paramethason, Fluocortolon, Deflazacort, Cloprednol und Fludrocortison oder Kombinationen davon, und eine Suchtdroge, ausgewählt aus der Gruppe umfassend Psychostimulantien, Halluzinogene und Entactogene, insbesondere Amphetamine, LSD und MDMA (Ecstasy), Nikotin, Cannabinoide, Kokain (inklusive "Crack").
   3. Verwendung gemäß 1 von Agonisten der Corticosteroidrezeptoren, ausgewählt aus den Verbindungen Cortisol, Cortison, Cortisonacetat, Corticosteron, Prednisolon, Prednison, Prednyliden, Methylprednisolon, Triamcinolon, Betamethason, Dexamethason, Paramethason, Fluocortolon, Deflazacort, Cloprednol und Fludrocortison oder Kombinationen davon und einem Opiat zur Herstellung eines Arzneimittels zur Therapie der durch Opiatsucht ausgelösten psychischen Abhängigkeit nach erfolgtem Entzug; und
   4. Verwendung gemäß 1 oder 3 eines Agonisten der Corticosteroidrezeptoren ausgewählt aus den Verbindungen Cortisol, Cortison, Cortisonacetat, Corticosteron, Prednisolon, Prednison, Prednyliden, Methylprednisolon, Triamcinolon, Betamethason, Dexamethason, Paramethason, Fluocortolon, Deflazacort, Cloprednol und Fludrocortison oder Kombinationen davon, und einem Opiat, zur Herstellung eines Arzneimittels zur Therapie der durch ein Opiat ausgelösten psychischen Abhängigkeit nach erfolgtem Entzug
      a) wobei der Agonist der Corticosteroidrezeptoren gefolgt von der gemeinsamen Verabreichung von dem Agonisten der Corticosteroidrezeptoren und dem Opiat zu verabreichen ist;
      b) wobei der Agonist der Corticosteroidrezeptoren gefolgt von gemeinsamer Verabreichung von Opiat und Agonisten der Corticosteroidrezeptoren, gefolgt von forcierter Verabreichung des Opiats zu verabreichen ist, oder
      c) wobei die Agonisten der Corticosteroidrezeptoren und Opiat gemeinsam zu geben sind, gefolgt von der forcierter Verabreichung des Opiats.

Im folgenden wird die mit der erfindungsgemäßen Verwendung und dem erfindungsgemäßen Arzneimittel durchführbare Therapie an einem etablierten Tiermodell näher erläutert.

Die Untersuchungen am Tiermodell begannen mit 96 männlichen Wistarratten, von denen schließlich 77 Tiere in die entscheidenden Therapieversuche eingingen. Die restlichen Tiere waren entweder vorzeitig gestorben oder dienten als unbehandelte Kontrollen für nachfolgende neurobiologische/neurochemische Untersuchungen. In der ersten Experimentierphase erhielt die Hälfte der Tiere das µ-agonistische Opioid Etonitazen (ETZ) im Heimkäfig zur freien Wahl (vierTrinkflüssigkeiten: Wasser, 2 mg ETZ/l; 4 mg ETZ/I; 8 mg ETZ/I), die andere Hälfte, blieb drogennaiv und erhielt nur Wasser als einzige Trinkflüssigkeit. Nach einigen Wochen der Eingewöhnung entwickelten die Ratten einen "kontrollierten" Konsum, d. h. sie nahmen - je nach Individualdisposition und situativen Faktoren - mehr oder weniger Opiat zu sich; es handelte sich jedoch stets um moderate Dosen (Mittelwert ± Standardabweichung: 9,5 ± 6,2 µg/kg/Tag).

Der Wahlversuch wurde solange fortgesetzt, bis der aus früheren Experimenten bekannte Bedarfsanstieg im Vorfeld einer Suchtentstehung einsetzte. In dieser Zeitspanne erhöhen die später als süchtig indentifizierten Tiere ihren Opiatkonsum von einer Woche auf die andere auf das vier- bis achtfache der ursprünglichen Dosis. Sobald der Bedarfsanstieg bei einem erheblichen Teil der Versuchstiere sichtbar war, wurde allen Tieren des betreffenden Versuchsansatzes das Opiat entzogen, die Tiere erhielten jetzt nur noch Wasser als einzige Trinklösung. Es wurden zwei zeitlich versetzte, voreinander unabhängige Versuchsansätze mit je 48 Tieren durchgeführt. Im ersten Ansatz begann der Entzug nach 40 Wochen, im zweiten Ansatz nach 44 Wochen.

Nach 15 Wochen Abstinenz (Wasser als einzige Trinkflüssigkeit) wurden alle Ratten (also auch die drogennaiven Tiere) einem Retest unterzogen, bei dem sie zwei Wochen lang die Wahl zwischen Wasser und Opiatlösungen (2 mg ETZ/I; 4 mg ETZ/I; 8 mg ETZ/I) hatten. Für zwei weitere Wochen wurden alle Opiatlösungen, nicht aber das alternativ dazu angebotene Wasser, mit dem bitter schmeckenden, für Ratten hochaversiven Chininhydrochlorid (0,1 g/l) vergällt. Alle zuvor drogennaiv gehaltenen Ratten (N = 29) senkten darauf ihre Opiateinnahme, ihre durchschnittliche Tagesdosis lag bei 6,2 ± 0,2 µg/kg/Tag (hier und im folgenden jeweils Mittelwerte ± SEM). Bei den drogenerfahrenen Tieren ließen sich zwei Gruppen streng voneinander unterscheiden. Die eine nahmen trotz Vergällung äußerst hohe ETZ-Dosen zu sich (90,6 ± 4,5 µg/kg/Tag, N = 20), die anderen reduzierten ihre Opiateinnahme auf einen ähnlichen Wert wie die zuvor drogennaiven Tieren (7,8 ± 0,7 µg/kg/Tag, N = 28). Die erstere Teilgruppe wurde - in Übereinstimmung mit früheren Resultaten - als "süchtig" klassifiziert. Alle diese Tiere hatten, im Gegensatz zu den restlichen, nicht süchtig gewordenen Ratten, nach dem kontrollierten Substanzkonsum einen Bedarfsanstieg gezeigt.

Wenn sich bei Ratten einmal eine Opiatsucht etabliert hat, dann verschwindet sie nicht spontan, sondern überdauert auch lange Abstinenzzeiten (Heyne, 1996). Unbehandelte opiatsüchtige Tiere behielten auch nach zwei weiteren Nach-Retests (7 bzw. 16 Wochen nach Ende des ersten Retests) ihre Sucht bei. Sie nahmen unter Vergällungsbedingungen durchschnittlich 105,2 ± 6,5 µg/kg/Tag zu sich. Nicht süchtig gewordene Ratten und zuvor drogennaive Tiere blieben ebenfalls langfristig stabil. In den Nach-Retests konsumierten sie unter Vergällungsbedingungen 6,6 ± 0,5 bzw. 6,5 ± 0,6 µg/kg/Tag. Die hohen Einnahmewerte der süchtigen Tiere gingen nicht auf verminderte Aversion bzw. sogar eine Präferenz gegenüber Chinin zurück, denn bei der Wahl zwischen Wasser und opiatfreien, chininvergällten Lösungen vermieden süchtige ebenso wie nicht-süchtige Tiere die Chininlösungen.

Zwei Wochen nach Ende des ersten Retest bzw. 11 Wochen danach (d. h. zwei Wochen nach Ende des ersten Nach-Retests) wurden Behandlungsversuche durchgeführt. Es wurden drei Therapiekonzepte getestet:
(A) Zweiwöchige Ketaminvorbehandlung (dreimal wöchentlich eine intraperitonale Injektion von 40 mg/kg S(+)-Ketamin), daran unmittelbar anschließend eine einwöchige forcierte Verabreichung des Opiats Etonitazen über das Trinkwasser (2 mg/l, mittlere Tagesdosis ± SEM: 115 ± 9 µg/kg/Tag).
(B) Zweiwöchige, forcierte Behandlung mit Corticosteron. Als einzige Trinkflüssigkeit war eine 250 mg/l Corticosteronlösung verfügbar, die mittlere Corticosteron-Tagesdosis betrug 13,4 ± 0,6 mg/kg/Tag.
(C) Eine kombinierte, dreiphasige Behandlung mit Corticosteron, dann ETZ + Corticosteron und zuletzt ETZ. Während der ersten Therapiewoche wurde eine 250 mg/l Corticosteronlösung als einzige Trinkflüssigkeit angeboten (Corticosteron-Tagesdosis: 11,7 ± 0,5 mg/kg/Tag). In der nächsten Woche enthielt diese einzig verfügbare Trinkflüssigkeit zusätzlich zum Corticosteron noch 2 mg ETZ/l. Die Tagesdosen betrugen nun 11,7 ± 0,6 mg/kg/Tag Corticosteron und 93 ± 5 µg/kg/Tag ETZ. In der dritten Woche wurde das Corticosteron abgesetzt, die Trinkflüssigkeit enthielt jetzt nur noch 2 mg/l ETZ (ETZ-Tagesdosis: 109 ± 5 µg/kg/Tag). Es sollte beachtet werden, daß die forcierten Tagesdosen nicht von denen abwichen, die ein süchtiges Tier freiwillig einnimmt.

Die Behandlungen (A) und (B) zeigten keinerlei Wirkung. In den Nach-Retests erwiesen sich die derart therapierten süchtigen Tiere nach wie vor als süchtig, sie unterschieden sich nicht von unbehandelten Artgenossen (110,4 ± 6,6 µg/kg/Tag nach Behandlung (A) und 100,1 ± 6,3 µg/kg/Tag nach Behandlung (B)). Dagegen hatte Behandlung (C) in allen acht Fällen, in denen sie bei zuvor süchtigen Ratten angewandt wurde, Erfolg. Die betreffenden Tiere nahmen unter Vergällungsbedingungen nur noch 11,2 ± 2,8 µg/kg/Tag ETZ zu sich. Sie hatten also ihre Sucht verloren. Dass dieser Behandlungserfolg nicht nur ein vorübergehender Effekt war, konnte in einem Nach-Test 11 Wochen nach Behandlung bestätigt werden. Die Ratten, die nach der Dreiphasentherapie ihre Sucht verloren hatten, erwiesen sich auch in diesem Test nach wie vor als nicht-süchtig. Ihre Einnahmewerte lagen bei 7,4 ± 0,6 µg/kg/Tag und unterschieden sich damit nicht von denen nicht süchtig gewordener bzw. zuvor drogennaiver Ratten.

Bei allen nicht süchtigen Tieren waren die Behandlungen wirkungslos, hier gab es weder spontane noch therapieverursachte Änderungen des Einnahmeverhaltens. In den Nach-Retests der diversen Gruppen ergaben sich Einnahmewerte zwischen 6.2 ± 0,8 und 8,8 ± 1,9 µg/kg/Tag, damit lagen die Dosen im Bereich des ersten Retests. Keines der nichtsüchtigen Tiere war süchtig geworden, keines änderte nach einer Behandlung sein Einnahmeverhalten.

Die Dreiphasentherapie hatte nicht nur - wie Anti-craving-Therapien - einen modulierenden Einfluss ausgeübt, sondern den einmal eingetretenen Kontrollverlust wieder rückgängig gemacht. Dies gelang weder mit einer Corticosteron-Behandlung noch mit einer forcierten Verabreichung der Suchtdroge allein. Weder Behandlung (A) noch Behandlung (B) waren erfolgreich, obwohl sie diese Behandlungskomponenten enthielten. Erst eine überlappende Sukzessivkombination beider Komponenten führte hier zum gewünschten Ergebnis. Alle erfindungsgemäß behandelten süchtigen Ratten wurden geheilt, alle nach anderen Therapiekonzepten behandelten oder unbehandelt gelassenen süchtigen Tiere behielten ihre Sucht bei. Es ist zwar zu vermuten, dass bei Wiederholung der Experimente mit einer noch größeren Zahl auch einige "Nonresponder" auftreten können, der vollständige Erfolg dieses Experimentes lässt aber erwarten, dass ein sehr hoher Prozentsatz der Individuen auf die Behandlung anspricht.

### Literaturübersicht

American Psychiatric Association (1994)
   Diagnostic and statistical manual of mental disorders (DSM IV), Washington D. C:
Brecht, J. G.; Poldrugo, F.; Schädlich, P. K. (1996)
   Die Krankheitskosten des Alkoholismus in der Bundesrepublik Deutschland, PharmacoEconomics 10: 484 - 493
Bühringer, G.; Künzel, J.; Spies, G. (1997)
   Methadon-Substitution bei Opiatabhängigen, In: H. Watzl und B. Rockstroh (Hrsg.) Abhängigkeit und Missbrauch von Alkohol und Drogen. Hogrefe Verlag, Göttingen, S. 249 - 264
Ehrenreich, H., Mangholz, A.; Schmitt, M.; Lieder, P.; Völkel, W.; Rüther, E.; Poser, W. (1997) OLITA: An alternative in the treatment of therapyresistant chronic alcoholics. First evaluation of a new approach. Eur. Arch. Psychiatry Clin. Neurosci. 247: 51 - 54
Finkbeiner, T.; Rösinger, C.; Gastpar, M. (1996)
   Grundlagen und praktische Durchführung der substitutionsgestützten Behandlung der Opiatabhängigkeit. In: K. Mann und G. Buchkremer (Hrsg.) Sucht-Grundlagen-Diagnostik-Therapie. Gustav Fischer Verlag, Stuttgart, Jena, New York S. 303 - 316
Heyne, A. (1996)
   The development of opiate addiction in the rat. Pharmacol. Biochem. Behav. 53; 11 - 25
Küfner, H. (1997)
   Behandlungsfaktoren bei Alkohol- und Drogenabhängigen. In: H. Watzl und B. Rockstroh (Hrsg.) Abhängigkeit und Missbrauch von Alkohol und Drogen. Hogrefe Verlag, Göttingen, S. 201 - 228
Küfner, H.; Feuerlein, W. (1989)
   In-patient treatment for alcoholism. A multi-center evaluation study. Springer Verlag, Berlin
Mann, K.; Mundle, G. (1996)
   Die pharmakologische Rückfallprophylaxe bei Alkoholabhängigen. Bedarf und Möglichkeiten. In: K. Mann und G. Buchkremer (Hrsg.) Sucht-Grundlagen-Diagnostik-Therapie. Gustav Fischer Verlag, Stuttgart, Jena, New York, S. 317 - 322
Rist, F. (1996)
   Therapiestudien mit Alkoholabhängigen. In: K. Mann und G. Buchkremer (Hrsg.) Sucht-Grundlagen-Diagnostik-Therapie. Gustav Fischer Verlag, Stuttgart, Jena, New York, S. 243 - 254
Roch, I.; Küfner, H.; Artz, J.; Böhmer, M.; Denis, A. (1992)
   Empirische Ergebnisse zum Therapieabbruch bei Drogenabhängigen: ein Literaturüberblick. Sucht 38: 305 - 322
Sass, H.; Soyka, M.; Mann, K.; Zieglgänsberger, W. (1996)
   Relapse revention in alcoholics with an anticraving drug treatment: first results of the Berlin study. Pharmacopsychiatry 27: 21 - 23
Schwoon, D. (1996)
   Nutzung professioneller Nachsorge und Selbsthilfegruppen durch Alkoholiker nach stationärer Kurzzeittherapie. In: K. Mann und G. Buchkremer (Hrsg.) Sucht-Grundlagen-Diagnostik-Therapie. Gustav Fischer Verlag, Stuttgart, Jena, New York, S. 281 - 288
Sobell, L. C.; Sobell, M. B.; Toneatto, T.; Leo, G. I. (1993)
   What triggers the resolution of alcohol problems without treatment? Alcohol Clin. Exp. Res. 17: 217 - 224
Soyka, M. (1997)
   Neuere medikamentöse Ansätze in der Alkoholismustherapie. In: H. Watzl und B. Rockstroh (Hrsg.) Abhängigkeit und Mißbrauch von Alkohol und Drogen. Hogrefe Verlag, Göttigen, S. 229 - 247
Stetter, F.; Axmann-Kremar (1996)
   Psychotherapeutische Motivationsarbeit bei Alkoholkranken in der Entgiftungsphase. In: K. Mann und G. Buchkremer (Hrsg.) Sucht-Grundlagen-Diagnostik-Therapie. Gustav Fischer Verlag, Stuttgart, Jena, New York, S. 255 - 264
Süß, H. M. (1995)
   Zur Wirksamkeit der Therapie bei Alkoholabhängigen: Ergebnisse einer Meta-Analyse. Psychol. Rundschau 46: 248 - 266
Veltrup, C.; Rohde, M.; Jacobi, M.; Koesters, C. (1995)
   Inanspruchnahme von Versorgungsangeboten durch Alkoholabhängige nach einer erweiterten Entzugsbehandlung. In: Ladewig, D. (Hrsg.) Drogen und Alkohol Nr. 8. ISPA-PRESS; Lausanne, S. 133 - 149 Whitworth, A. B.; Fischer, E.; Lesch, O. M.; Nimmerrichter, A.; Oberbauer, H.; Platz, T.; Potgierer, A.; Walter, H.; Fleischhacker, C. (1996) Comparison of acamprosate and placebo in the long term treatment of alcohol dependence. Lancet 347: 1438 - 1442
Wieser, S.; Kunad, E. (1965)
   Katamnestische Studien beim chronischen Alkoholismus und zur Folge von Sozialprozessen bei Alkoholikern. Nervenarzt 36: 477 - 483
Wolffgramm, J. (1995)
   Abhängigkeitsentwicklung im Tiermodell. Z. Klin. Psychol. 24: 107 - 117
Wolffgramm, J.; Heyne, A. (1995)
   From controlled drug intake to loss of control: the irreversible development of drug addiction in the rat. Behav. Brain Res. 70: 77 - 94

## Patentansprüche

1. Verwendung von Agonisten der Corticosteroidrezeptoren, ausgewählt aus den Verbindungen Cortisol, Cortison, Cortisonacetat, Corticosteron, Prednisolon, Prednison, Prednyliden, Methylprednisolon, Triamcinolon, Betamethason, Dexamethason, Paramethason, Fluocortolon, Deflazacort, Cloprednol und Fludrocortison oder Kombinationen davon, und einer Suchtdroge, ausgewählt aus der Gruppe umfassend Opiate, Psychostimulantien, Halluzinogene und Entactogene, insbesondere Amphetamine, LSD und MDMA (Ecstasy), Nikotin, Cannabinoide, Kokain (inklusive "Crack") zur Herstellung eines Arzneimittels zur Therapie der durch die Suchtdroge ausgelösten psychischen Abhängigkeit nach erfolgtem Entzug.

2. Arzneimittel enthaltend einen Agonisten der Corticosteroidrezeptoren ausgewählt aus den Verbindungen Cortisol, Cortison, Cortisonacetat, Corticosteron, Prednisolon, Prednison, Prednyliden, Methylprednisolon, Triamcinolon, Betamethason, Dexamethason, Paramethason, Fluocortolon, Deflazacort, Cloprednol und Fludrocortison oder Kombinationen davon, und eine Suchtdroge, ausgewählt aus der Gruppe umfassend Psychostimulantien, Halluzinogene und Entactogene, insbesondere Amphetamine, LSD und MDMA (Ecstasy), Nikotin, Cannabinoide, Kokain (inklusive "Crack").

3. Verwendung von Agonisten der Corticosteroidrezeptoren und einem Opiat gemäß Anspruch 1 zur Herstellung eines Arzneimittels zur Therapie der durch Opiatsucht ausgelösten psychischen Abhängigkeit nach erfolgtem Entzug.

4. Verwendung eines Agonisten der Corticosteroidrezeptoren und einem Opiat, gemäß Anspruch 1, zur Herstellung eines Arzneimittels zur Therapie der durch ein Opiat ausgelösten psychischen Abhängigkeit nach erfolgtem Entzug,
a) wobei der Agonist der Corticosteroidrezeptoren gefolgt von der gemeinsamen Verabreichung von dem Agonisten der Corticosteroidrezeptoren und dem Opiat zu verabreichen ist;
b) wobei der Agonist der Corticosteroidrezeptoren gefolgt von gemeinsamer Verabreichung von Opiat und Agonisten der Corticosteroidrezeptoren, gefolgt von forcierter Verabreichung des Opiats zu verabreichen ist, oder
c) wobei die Agonisten der Corticosteroidrezeptoren und Opiat gemeinsam zu geben sind, gefolgt von der forcierter Verabreichung des Opiats.

## Claims

1. Use of agonists of the corticosteroid receptors selected from the compounds cortisole, cortisone, cortisone acetate, corticosterone, prednisolone, prednisone, prednylidene, methylprednisolone, triamcinolone, betamethasone, dexamethasone, paramethasone, fluocortolone, deflazacort, cloprednol and fludrocortisone or combinations thereof, and an addictive drug selected from the group comprising opiates, psychostimulants, hallucinogens and entactogens, particularly amphetamines, LSD and MDMA (ecstasy), nicotine, cannabinoids, cocaine (including "crack") for the preparation of a pharmaceutical composition for the therapy of the psychological dependency caused by the addictive drug after withdrawal.

2. Pharmaceutical composition containing an agonist of the corticosteroid receptors selected from the compounds cortisole, cortisone, cortisone acetate, corticosterone, prednisolone, prednisone, prednylidene, methylprednisolone, triamcinolone, betamethasone, dexamethasone, paramethasone, fluocortolone, deflazacort, cloprednol and fludrocortisone or combinations thereof, and an addictive drug selected from the group comprising psychostimulants, hallucinogens and entactogens, particularly amphetamines, LSD and MDMA (ecstasy), nicotine, cannabinoids, cocaine (including "crack").

3. Use of agonists of the corticosteroid receptors and an opiate according to claim 1 for the preparation of a pharmaceutical composition for the therapy of the psychological dependency caused by opiate addiction after withdrawal.

4. Use of an agonist of the corticosteroid receptors and an opiate according to claim 1 for the preparation of a pharmaceutical composition for the therapy of the psychological dependency caused by opiate addiction after withdrawal,
a) wherein the agonist of the corticosteroid receptors is to be administered followed by the joint administration of the agonist of the corticosteroid receptors and the opiate,
b) wherein the agonist of the corticosteroid receptors is to be administered followed by the joint administration of opiate and agonist of the corticosteroid receptors, followed by the forced administration of the opiate, or
c) wherein the agonists of the corticosteroid receptors and the opiate are to be jointly administered followed by the forced administration of the opiate.

## Revendications

1. Utilisation d'agonistes des récepteurs de corticostéroïdes, sélectionnés parmi les composés cortisol, cortisone, acétate de cortisone, corticostérone, prednisolone, prednisone, prédnylidène, méthylprednisolone, triamcinolone, bétaméthasone, dexaméthasone, paraméthasone, fluocortolone, deflazacort, cloprednole et fludrocortisone ou des mélanges de ceux-ci, et d'une drogue de pharmacodépendance, choisie parmi le groupe comprenant les opiacés, les psychostimulants, les hallucinogènes et les entactogènes, en particulier les amphétamines, le LSD et le MDMA (ecstasy), la nicotine, les cannabinoïdes, la cocaïne (y compris le crack) pour la fabrication d'un médicament destiné au traitement de la dépendance psychique déclenchée par la drogue de pharmacodépendance après la désintoxication effectuée.

2. Médicament contenant un agoniste des récepteurs de corticostéroïdes, sélectionné parmi les composés cortisol, cortisone, acétate de cortisone, corticostérone, prednisolone, prednisone, prédnylidène, méthylprednisolone, triamcinolone, bétaméthasone, dexaméthasone, paraméthasone, fluocortolone, deflazacort, cloprednole et fludrocortisone ou des mélanges de ceux-ci, et une drogue de pharmacodépendance, choisie parmi le groupe comprenant les opiacés, les psychostimulants, les hallucinogènes et les entactogènes, en particulier les amphétamines, le LSD et le MDMA (ecstasy), la nicotine, les cannabinoïdes, la cocaïne (y compris le crack).

3. Utilisation d'agonistes des récepteurs de corticostéroïdes et d'une substance opiacée selon la revendication 1, pour la fabrication d'un médicament destiné au traitement de la dépendance psychique déclenchée par la dépendance à la substance opiacée après la désintoxication effectuée.

4. Utilisation d'un agoniste des récepteurs de corticostéroïdes et d'une substance opiacée selon la revendication 1, pour la fabrication d'un médicament destiné au traitement de la dépendance psychique déclenchée par une substance opiacée après la désintoxication effectuée,
a) dans laquelle l'agoniste des récepteurs de corticostéroïdes est à administrer, suivi de l'administration commune de la substance opiacée et de l'agoniste des récepteurs de corticostéroïdes ;
b) dans laquelle l'agoniste des récepteurs de corticostéroïdes est à administrer, suivi de l'administration commune de la substance opiacée et de l'agoniste des récepteurs de corticostéroïdes, suivi de l'administration forcée de la substance opiacée, ou
c) dans laquelle les agonistes des récepteurs de corticostéroïdes et la substance opiacée sont à donner ensemble, suivi de l'administration forcée de la substance opiacée.
